# EUROPÄISCHE PATENTSCHRIFT

(11) **EP 0 866 047 B1**
(45) Veröffentlichungstag und Bekanntmachung des Hinweises auf die Patenterteilung: **22.08.2001**
(21) Anmeldenummer: 98102135.5
(22) Anmeldetag: 07.02.1998
(51) Int. Cl.: C07C 29/38, C07C 67/31, C07C 69/34, C07C 253/30, C07C 255/16

(54) **Verfahren zur Herstellung von Bishydroxymethylverbindungen**
Method for the preparation of bishydroxymethyl compounds
Procédé de préparation des composés bishydroxyméthylés

(30) Priorität: 21.03.1997 DE 19711762
(43) Veröffentlichungstag der Anmeldung: 23.09.1998
(73) Patentinhaber: Degussa AG, 40474 Düsseldorf (DE)
(72) Erfinder: Bauer, Frank, Dr., 53127 Bonn (DE)

(56) Entgegenhaltungen:
- A.E. ARDIS, ET AL.: "Vinylidene cyanide. I" JOURNAL OF THE AMERICAN CHEMICAL SOCIETY, Bd. 72, Nr. 3, 23.März 1950, WASHINGTON, DC, US, Seiten 1305-1307, XP002071832
- CHEMICAL ABSTRACTS, vol. 76, no. 23, 5.Juni 1972 Columbus, Ohio, US; abstract no. 139905m, S. KUNICHIKA, ET AL.: "Reaction of malonic esters with formaldehyde" Seite 398; XP002071834 & NIPPON KAGAKU KAISHI, Nr. 3, 1972, Seiten 596-598,
- A.R. DERZHINSKII, ET AL.: "Organic sulphur compounds containing functional groups, communication 5. Synthesis of alpha,beta-epoxyalkyl sulphones" BULLETIN OF THE ACADEMY OF SCIENCES OF THE USSR, DIVISION OF CHEMICAL SCIENCE, Bd. 31, Nr. 6, Pt. 2, Juni 1982, NEW YORK, US, Seiten 1225-1231, XP002071831
- C. TSCHIERSKE, ET AL.: "Synthesis and mesomorphic properties of axially cyano-substituted 1,3-dioxane-5-carboxylates" MOLECULAR CRYSTALS AND LIQUID CRYSTALS, Bd. 177, 1989, READING, GB, Seiten 113-121, XP002071833

## Beschreibung

Die vorliegende Erfindung betrifft ein Verfahren zur Herstellung von Bishydroxymethylverbindungen der allgemeinen Formel 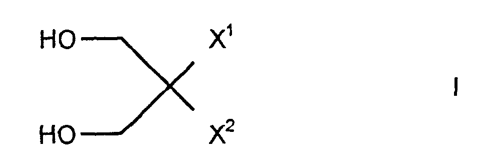 in der X¹ und X² unabhängig voneinander die Gruppen ―COOR, -CONR₂, -CN, -NO₂, -C(OR)=NR oder ―COR bedeuten, wobei R für Wasserstoff, Alkyl, Aralkyl, Aryl oder Cycloalkyl mit jeweils bis zu 12 Kohlenstoffatomen steht, mit der Maßgabe, dass (i) nicht beide Substituenten X¹ und X² gleichzeitig -COOH bedeuten können und (ii), wenn beide Substituenten X¹ und X² gleichzeitig ―CO-R bedeuten, die beiden Substituenten R zusammen auch einen Alkylenrest mit 3 bis 9 Kohlenstoffatomen bedeuten können.

Bishydroxymethylverbindungen der allgemeinen Formel I sind als organische Zwischenprodukte, z. B. als Vorstufen für UV-Stabilisatoren oder Röntgenkontrastmittel, von kommerziellem Interesse (siehe z. B. EP-A2 0 220 034).

Literaturbekannte Verfahren zur Herstellung von Verbindungen der allgemeinen Formel l gehen üblicherweise von den entsprechenden C-H-aciden Verbindungen der allgemeinen Formel II 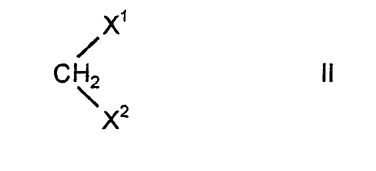 aus, in der X¹ und X² wie zuvor definiert sind. Diese Verbindungen werden in wässrigen Reaktionsmedien im Sinne einer Knoevenagel-Kondensation umgesetzt.

Wird die Reaktion - wie für den technisch bedeutenden Bishydroxymethylmalonsäurediethylester [siehe (a) Gault, Roesch; Bull. Soc. Chim. Fr. (5)**4** (1937), 1411; sowie (b) Lenz, R.W.; Saunders, K.; Balakrishnan, T.; Macromolecules **12,** 3 (1979), 392; und (c) Chang, H.M. et al.; J. Med. Chem. **34** (1991) 5, 1675] und den Bishydroxymethylcyanessigsäureethylester [siehe Tschierske, C.; Vorbrodt, H.-M.; Kresse, H., Zaschke, H.; Mol. Cryst. Liq. **177** (1989), 113] beschrieben - in wässriger Lösung unter Verwendung von KHCO₃, K₂CO₃ oder NaOH als Katalysator durchgeführt, so beobachtet man zwar im Vergleich zur sauren Katalyse sehr gute Selektivitäten. Für die Realisierung des Verfahrens im technischen Maßstab ergeben sich jedoch eine Reihe von erheblichen Nachteilen, welche sich besonders aus der erforderlichen Abtrennung des Wassers beziehungsweise der Produktisolierung ergeben.

So verbietet sich eine destillative Isolierung in der Regel wegen erheblicher Produktverluste durch thermische Zersetzungen. Die dabei entstehenden gasförmigen Zersetzungsprodukte (Welch, J., J. Chem. Soc. **1930** 258) führen zudem dazu, dass kein stabiles Vakuum erhalten werden kann. Eine extraktive Isolierung der Bishydroxymethylverbindungen aus der wässrigen Reaktionslösung hat sich insbesondere bei den technisch bedeutsamen Bishydroxymethylmalonsäureestern mit R¹, R² = -COOCH₃ oder -COOC₂H₅ als sehr aufwendig erwiesen, da diese recht gut wasserlöslich sind.

Zwar lässt sich nach Organic Syntheses, **40,** 27 der Bishydroxymethylmalonsäurediethylester aus der wässrigen Reaktionslösung durch Aussalzen mit Ammoniumchlorid extrahieren, die gesamte Stickstoff-Fracht verbleibt jedoch hierbei in der wässrigen Phase und macht eine aufwendige Entsorgung notwendig. Mehrstufige Extraktionen mit Wasser führen zudem dazu, dass neben den gewünschten Produkten auch erhebliche Mengen an Wasser in die organische Phase übergehen. Dies wirkt sich insbesondere dann negativ aus, wenn eine Weiterverarbeitung der Bishydroxymethylmalonsäureester unter wasserfreien Bedingungen erfolgen soll.

Da es sich bei den Bishydroxymethylverbindungen der allgemeinen Formel I durchweg um sehr polare und damit gut wasserlösliche sowie thermisch nicht sehr stabile Substanzen handelt, treten ähnliche Probleme auch bei den übrigen Bishydroxymethylverbindungen der allgemeinen Formel I auf.

Unabhängig von der Art der gewählten Methode zur Abtrennung der Bishydroxymethylverbindungen der allgemeinen Formel I fallen bei deren Herstellung Abwasserströme an, welche zumindest nicht vollkommen frei von Formaldehyd sind und daher nicht ohne weiteres entsorgt werden können. Dies ist praktisch prohibitiv für eine Herstellung von Bishydroxymethylverbindungen der allgemeinen Formel I im technischen Maßstab.

Hinzu kommt, dass die Bishydroxymethylverbindungen der allgemeinen Formel I als typische organische Zwischenprodukte recht oft in wasserfreier Form benötigt werden, um bei Folgereaktionen, z. B. der Umsetzung zu 2-substituierten 1,3-Dioxan-5,5-dicarbonsäurederivaten gemäß DE-A-197 11 758, hohe Ausbeuten zu erzielen bzw. eine möglichst einfache Verfahrensweise zu erlauben.

Es sind auch Verfahren bekannt geworden, die wasserfrei arbeiten. So wurde die Herstellung von Bishydroxymethylmalonsäurediethylester durch Umsetzung von Malonsäurediethylester mit p-Formaldehyd in Essigsäure in Gegenwart von Kupferacetat bei vergleichsweise hohen Temperaturen beschrieben (Japan. Patentanm. 072564 vom 30.05.1980) bei der natürlich die aufwendige Abtrennung von Wasser entfällt. Es fallen jedoch O-acetylierte und nicht-acetylierte Verbindungen in einem nur schwierig zu trennenden Gemisch an, und die Selektivität für die gewünschte nicht-acetylierte Verbindung ist gering [Kunichika, S.; Oka, S.; Sugiyama, T.; Nakamura, K.; Fukui, K.; Nippon Kagaku Kaishi **3** (1972), 596]. Außerdem führen Reaktionstemperaturen oberhalb von 50 °C, die für eine befriedigende Raum-Zeit-Ausbeute erforderlich sind, zu erheblichen Produktverlusten durch thermische Zersetzung sowie durch Bildung von Methylenmalonsäurediethylester.

Weiterhin ist bekannt, dass Malonsäuredinitril in Eisessig in Gegenwart von Kaliumacetat mit gasförmigem Formaldehyd bei 100 °C zu Bismethylolmalonsäuredinitril umgesetzt werden kann [Ardis, A.E.; Averill, S.J.; Gilbert, H.; Miller, F.F.; Schmidt, R.F.; Stewart, F.D.; und Trumbull, H.L.; J. Am. Chem. Soc., **72** (1950), 1305]. Dabei beträgt die Ausbeute jedoch nur 24 % d. Th.

Es bestand also ein Bedarf an einem Verfahren zur Bishydroxymethylierung von Verbindungen der allgemeinen Formel II, welches hohe Ausbeuten an den entsprechenden Bishydroxymethylverbindungen der allgemeinen Formel I ergibt, die in reiner Form oder aber im Reaktionsgemisch mit hohen Ausbeuten weiter umgesetzt werden können und bei dem keine Ammonium- oder andere Salze und/oder Formaldehyd enthaltende Abwässer anfallen.

Dementsprechend wurde nun überraschend gefunden, dass man Bishydroxymethylverbindungen der allgemeinen Formel 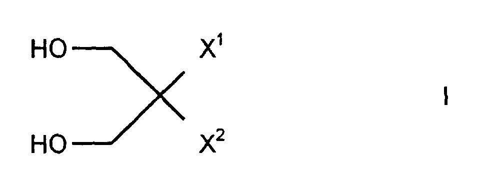 in der X¹ und X² unabhängig voneinander -COOR, -CONR₂, -CN, -NO₂, -C(OR)=NR oder ―COR bedeuten, wobei R für Wasserstoff, Alkyl, Aralkyl, Aryl oder Cycloalkyl mit jeweils bis zu 12 Kohlenstoffatomen steht, mit der Maßgabe, dass (i) nicht beide Substituenten X¹ und X² gleichzeitig -COOH bedeuten können und (ii), wenn beide Substituenten X¹ und X² gleichzeitig-CO-R bedeuten, die beiden Substituenten R zusammen auch einen Alkylenrest mit 3 bis 9 Kohlenstoffatomen bedeuten können, durch Umsetzung von C-H-aciden Verbindungen der allgemeinen Formel 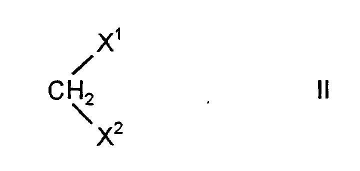 in der X¹ und X² wie zuvor definiert sind, mit Formaldehyd vorteilhaft erhält, wenn man Formaldehyd in Form von p-Formaldehyd verwendet und die Reaktion in einem wasserfreiem Medium, wobei Essigsäure ausgeschlossen wird, durchführt.

In besonders bevorzugten Bishydroxymethylverbindungen der allgemeinen Formel I bzw. C-H-aciden Verbindungen der allgemeinen Formel II stehen X¹ und X² unabhängig voneinander für -COOR oder -CN, wobei R einen C₁-C₄-Alkylrest bedeutet.

Von den geeigneten C-H-aciden Verbindungen II seien beispielsweise erwähnt: Malonsäuredimethylester, Malonsäurediethylester, Malonsäuredi-n-propylester, Malonsäurediisobutylester, Malonsäuredibenzylester, Malonsäuredi-2-ethylhexylester, N,N-Dimethylcarbamidoessigsäureethylester, N,N,N',N'-Tetramethylmalonsäurediamid, Malonsäuredinitril, Cyanessigsäureethylester, Cyanessigsäure-n-butylester, Nitroessigsäureethylester, Acetylaceton und Cyclododeca-1,3-dion.

Formaldehyd wendet man in Form des festen p-Formaldehyds an. Die C-C-acide Verbindung II und der Formaldehyd können in stöchiometrischen Mengen, d. h. im Molverhältnis 2 : 1 eingesetzt werden, jedoch schadet ein geringer Überschuss der einen oder anderen Komponente, insbesondere an Formaldehyd, im Allgemeinen nicht.

Vorteilhaft führt man das erfindungsgemäße Verfahren in Gegenwart eines basischen Katalysators durch. Als solche eignen sich vor allem Metallhydroxide, -carbonate, -hydrogencarbonate, basische lonenaustauscher oder Alkoholate. Bevorzugt werden Alkalimetallhydroxide, -carbonate, -hydrogencarbonate und -alkoholate sowie basische lonenaustauscher mit quaternären Ammoniumgruppen. Der Katalysator kann fest (d. h. im Reaktionsgemisch suspendiert oder als Trägerkatalysator fest angeordnet) oder im Reaktionsgemisch gelöst sein. Als gelöste Katalysatoren eignen sich vor allem die Alkalialkoholate, insbesondere wenn, wie in der Folge erläutert, ein Alkanol als Lösungsmittel mitverwendet wird. Sie werden zweckmäßig in Mengen von 0,01 bis 5,0 Gew.-%, insbesondere von 0,01 bis 2,0 Gew.-%, bezogen auf die C-H-acide Verbindung der allgemeinen Formel II, eingesetzt. Brauchbare feste, im Reaktionsgemisch suspendierte oder gegebenenfalls zum Teil gelöste Katalysatoren sind u. a. Kaliumhydrogencarbonat, Kaliumcarbonat, Natriumcarbonat und Natriumhydrogencarbonat. Deren Menge beträgt zweckmäßig 0,01 bis 10 Gew.-%, bezogen auf die C-H-acide Verbindung der allgemeinen Formel II.

Setzt man saure lonenaustauscher ein, so kann deren Menge beträchtlich höher liegen. Das ist z. B. der Fall, wenn man das Ausgangsgemisch über fest angeordnete saure lonenaustauscher laufen lässt.

Die Verwendung eines Lösungsmittels ist nicht zwingend erforderlich, die Reaktionsführung sowie die Handhabung der Reaktionsmischung werden jedoch durch die Verwendung eines Lösungsmittels vereinfacht. Als Lösungsmittel kommen prinzipiell alle Lösungsmittel in Frage, welche unter den gewählten Reaktionsbedingungen nicht mit den Edukten II und Formaldehyd reagieren. In der Praxis hat sich jedoch die Verwendung von polaren Lösungsmitteln, insbesondere von Alkanolen, als vorteilhaft erwiesen. Natürlich kann man auch Lösungsmittelgemische einsetzen. Weniger empfehlenswert sind saure Lösungsmittel, Essigsäure wird als Lösungsmittel ausgeschlossen. Der Anteil des Lösungsmittels oder Lösungsmittelgemisches im Reaktionsgemisch beträgt am Ende der Reaktion im Allgemeinen 10 bis 80,0 Gew.-%. Man kann die Reaktion natürlich auch in noch größerer Verdünnung durch Lösungsmittel durchführen, doch leidet dann die Raum-Zeit-Ausbeute.

Es ist ein wesentlicher Vorzug des Verfahrens nach der Erfindung, dass die Reaktion in wasserfreiem Medium durchgeführt werden kann. Wie erwähnt, werden dadurch die Abwasserproblematik und die Schwierigkeiten bei der Gewinnung des Produktes aus wässrigen Gemischen vermieden. Erhebliche Vorteile ergeben sich gegenüber den Verfahren in wässrigem Medium aber auch dann, wenn das Reaktionsgemisch nur im Wesentlichen wasserfrei ist, d. h. lediglich kleine Mengen an Wasser, z. B. bis zu 5,0 Gew.-%, enthält. Solche kleinen Mengen können u. a. durch das Lösungsmittel eingeschleppt werden. Auch eine Reaktion unter im Wesentlichen wasserfreien Bedingungen fällt in den Bereich der vorliegenden Erfindung.

Das erfindungsgemäße Verfahren wird bei Reaktionstemperaturen von ―30 bis 200 °C durchgeführt, wobei die Reaktionszeiten den Reaktionstemperaturen so angepasst werden, dass eine merkliche Zersetzung der Bishydroxymethylverbindungen der allgemeinen Formel I praktisch ausgeschlossen ist. Bei absatzweisen Ausführungsformen des erfindungsgemäßen Verfahrens verläuft die Bishydroxymethylierung bei Temperaturen unterhalb von ―30 °C im Allgemeinen mit zu geringen Reaktionsgeschwindigkeiten, während oberhalb von 120 °C nicht selten starke Zersetzungsreaktionen auftreten. Ausreichend schnelle Umsetzungen und hohe Selektivitäten erzielt man im Temperaturbereich von 0 bis 60 °C und insbesondere im Temperaturbereich von 15 bis 30 °C. Demgemäß werden diese Temperaturbereiche besonders bevorzugt. Bei Anwendung von p-Formaldehyd begünstigt eine Temperaturerhöhung gegen Ende der Reaktion hohe Raum-Zeit-Ausbeuten und hohe Umsätze. Die Reaktionszeiten betragen bei der absatzweisen Variante des Verfahrens im Allgemeinen 0,5 bis 5,0 Stunden.

Bei der kontinuierlichen Herstellung der Bishydroxymethylverbindungen der allgemeinen Formel I sind - bei gleichzeitiger, gegebenenfalls drastischer Verkürzung der Reaktionszeiten, Temperaturen bis 200 °C und höher möglich. Geeignete Kombinationen von Reaktionstemperatur und Reaktionszeit lassen sich für ein gegebenes Reaktionssystem durch orientierende Versuche unschwer ermitteln.

Als vorteilhafte Ausführungsform hat sich in der Praxis eine Dosierung der C-H-aciden Verbindung II zu einer Suspension von p-Formaldehyd in einem inerten Lösungsmittel oder Lösungsmittelgemisch bewährt. Neben vergleichsweise hohen Selektivitäten gewährleistet diese Vorgehensweise, dass die frei werdende Reaktionswärme sicher abgeführt werden kann. Das Produkt kann vielfach ohne Abtrennung des Katalysators weiterverarbeitet werden. Natürlich kann der Katalysator auch abgetrennt werden, z. B. durch Filtration, wenn der Katalysator ein Feststoff ist, oder durch Neutralisierung mit einer Säure und Abfiltrieren des entstehenden Salzes, wenn man einen gelösten Katalysator verwendet hatte. Im letzteren Falle wird zweckmäßig ein möglichst unpolares Solvens zugesetzt, in dem die Bishydroxymethylverbindungen der allgemeinen Formel I jedoch löslich sein müssen, da die Letzteren im Allgemeinen polare Feststoffe sind. Als Beispiele für geeignete vergleichsweise unpolare Solventien seien Diisopropylether, Methyl-tert.-butylether und Ethylacetat genannt. Nach der Abtrennung des gegebenenfalls neutralisierten Katalysators kann man das Lösungsmittel entfernen, gegebenenfalls unter vermindertem Druck, und erhält als Rückstand die Bishydroxymethylverbindung der allgemeinen Formel I.

Die nach dem erfindungsgemäßen Verfahren in wasserfreier bzw. im Wesentlichen wasserfreier Form erhaltenen Bishydroxymethylverbindungen der allgemeinen Formel 1 können nach Abtrennung des Lösungsmittels als solche oder in Form des nicht aufgearbeiteten Reaktionsgemisches direkt in weiteren Synthesen eingesetzt werden, welche ein wasserfreies oder praktisch wasserfreies Edukt erfordern. Es ist ein besonderer Vorzug des Verfahrens nach der Erfindung, dass die Bishydroxymethylverbindungen der allgemeinen Formel I in dem Reaktionsgemisch, in dem sie anfallen, ohne Isolierung oder Reinigung weiter umgesetzt werden können und dann kaum schlechtere Ausbeuten an Folgeprodukt ergeben als die isolierten Bishydroxymethylverbindungen der allgemeinen Formel I.

Die folgenden Beispiele sollen das Verfahren nach der Erfindung weiter erläutern, nicht aber dessen Anwendungsbereich limitieren.

### Beispiel 1

### Bishydroxymethylmalonsäurediethylester

Zu einer bei 25 bis 30 °C gerührten Suspension aus 60,0 g p-Formaldehyd (2,0 Mol) und 0,25 g Natriumethanolat in 40,0 g Ethanol werden innerhalb von 1,75 Stunden 160,2 g Malonsäurediethylester (1,0 Mol) dosiert. Man ließ 2 Stunden bei 50 °C nachreagieren, kühlte auf 45 °C ab und fügte dann 0,36 g konzentrierte Schwefelsäure zu. Nach dem Abkühlen auf Raumtemperatur wurde der ausgefallene weiße Niederschlag über eine Glasfilternutsche abgesaugt. Das erhaltene farblose Filtrat wurde im Wasserstrahlvakuum (16 mm) bei einer Badtemperatur von 60 °C von Leichtsiedern befreit, wobei 121,2 g (96 % d. Th. gemäß Auswaage) Reaktionsprodukt in Form eines klaren farblosen Öls verblieben. Die gaschromatographisch bestimmte Reinheit des Materials wurde nach Silylieren zu 85 FID-Flächenprozent bestimmt.

### Beispiel 2

### Bishydroxymethylcyanessigsäureethylester

Zu einer bei 0 bis 10°C gerührten Mischung aus 62,0 g p-Formaldehyd (2,1 Mol), 200 g Ethanol und 0,25 g Natriumethanolat wurden innerhalb von 2,0 Stunden 113,0 g Cyanessigsäureethylester (1,0 Mol) dosiert. Die Reaktionsmischung wurde anschließend noch 1 Stunde bei 10°C gerührt, bevor man sie innerhalb von 10 Minuten auf Raumtemperatur erwärmte.

Die so erhaltene farblose klare Lösung wurde am Rotationsverdampfer bei einem Vakuum von 8 mbar vom Lösungsmittel befreit, wobei 175,1 g Bishydroxymethylcyanessigsäureethylester mit einer gaschromatographisch bestimmten Reinheit von 71,2 FID-Flächenprozent (silylierte Probe) erhalten wurden. Unter Berücksichtigung der Produktreinheit betrug die Ausbeute 72,9 % d. Th., bezogen auf eingesetzten Cyanessigsäureethylester.

## Patentansprüche

1. Verfahren zur Herstellung von Bishydroxymethylverbindungen der allgemeinen Formel 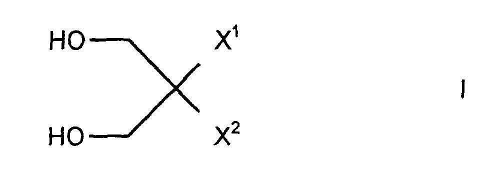
in der X¹ und X² unabhängig voneinander die Gruppen ―COOR, -CONR₂, -CN, -NO₂, -C(OR)=NR oder ―COR bedeuten, wobei R für Wasserstoff, Alkyl, Aralkyl, Aryl oder Cycloalkyl mit jeweils bis zu 12 Kohlenstoffatomen steht, mit der Maßgabe, dass (i) nicht beide Substituenten X¹ und X² gleichzeitig ―COOH bedeuten können und (ii), wenn beide Substituenten X¹ und X² gleichzeitig ―CO-R bedeuten, die beiden Substituenten R zusammen auch einen Alkylenrest mit 3 bis 9 Kohlenstoffatomen bedeuten können, durch Umsetzung von C-H-aciden Verbindungen der allgemeinen Formel 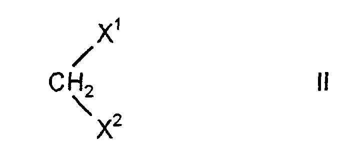
in der X¹ und X² wie zuvor definiert sind, mit Formaldehyd,
dadurch gekennzeichnet,
dass man Formaldehyd in Form von p-Formaldehyd verwendet und die Reaktion in wasserfreiem Medium, wobei Essigsäure ausgeschlossen ist, durchführt.

2. Verfahren nach Anspruch 1,
dadurch gekennzeichnet,
dass X¹ und X² unabhängig voneinander für ―COOR oder ―CN stehen, wobei R einen C₁-C₄-Alkylrest bedeutet.

3. Verfahren nach einem der Ansprüche 1 bis 2,
dadurch gekennzeichnet,
dass man ein polares Lösungsmittel mitverwendet.

4. Verfahren nach Anspruch 3,
dadurch gekennzeichnet,
dass das polare Lösungsmittel ein C₁- bis C₄-Alkohol ist.

5. Verfahren nach einem der Ansprüche 1 bis 4,
dadurch gekennzeichnet,
dass man einen basischen Katalysator mitverwendet.

6. Verfahren nach Anspruch 5,
dadurch gekennzeichnet,
dass der basische Katalysator ein Metallhydroxid, -carbonat, -hydrogencarbonat, ein basischer lonenaustauscher oder ein Alkoholat ist.

7. Verfahren nach Anspruch 5,
dadurch gekennzeichnet,
dass der basische Katalysator ein Alkalimetallhydroxid, -carbonat, -hydrogencarbonat oder -alkoholat ist.

8. Verfahren nach einem der Ansprüche 1 bis 7,
dadurch gekennzeichnet,
dass das Verfahren absatzweise durchgeführt wird und die Reaktionstemperatur -30 bis 120 °C beträgt.

9. Verfahren nach einem der Ansprüche 1 bis 7,
dadurch gekennzeichnet,
dass das Verfahren absatzweise durchgeführt wird und die Reaktionstemperatur 0 bis 60 °C beträgt.

10. Verfahren nach einem der Ansprüche 1 bis 7,
dadurch gekennzeichnet,
dass das Verfahren kontinuierlich durchgeführt wird und die Reaktionstemperatur bis zu 200 °C beträgt.

11. Verfahren nach einem der Ansprüche 1 bis 9,
dadurch gekennzeichnet,
dass das Verfahren absatzweise durchgeführt wird, indem man die C-H-acide Verbindung der allgemeinen Formel II zu einer Suspension von p-Formaldehyd in einem Lösungsmittel oder Lösungsmittelgemisch dosiert.

12. Verfahren nach Anspruch 10,
dadurch gekennzeichnet,
dass man nach Beendigung der Reaktion den festen Katalysator abtrennt oder den gelösten Katalysator neutralisiert und das entstehende Salz abtrennt.

13. Verfahren nach einem der Ansprüche 1 bis 12,
dadurch gekennzeichnet,
dass die Bishydroxymethylverbindung der allgemeinen Formel I in dem Reaktionsgemisch, in dem sie anfällt, ohne Isolierung weiter umgesetzt wird.

## Claims

1. A process for preparing bis(hydroxymethyl) compounds of the general formula 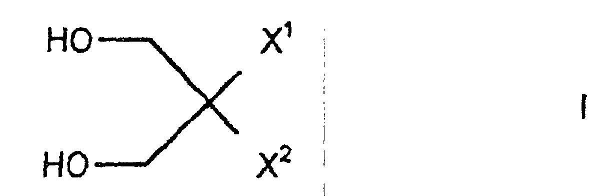 where X¹ and X², independently of one another, represent the groups -COOR, -CONR₂, -CN, -NO₂, -C(OR)=NR or -COR, R being hydrogen, alkyl, aralkyl, aryl or cycloalkyl each having up to 12 carbon atoms, with the proviso that (i) the two substituents X¹ and X² cannot simultaneously represent -COOH and (ii), if the two substituents X¹ and X² simultaneously represent -CO-R, the two substituents R together may also represent an alkylene radical having from 3 to 9 carbon atoms, by reacting C-H-acidic compounds of the general formula 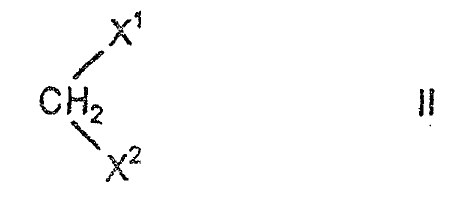 where X¹ and X² are as defined above, with formaldehyde, characterised in that formaldehyde is employed in the form of paraformaldehyde and the reaction is carried out in an anhydrous medium, with acetic acid being excluded.

2. A process as claimed in 1, characterised in that X¹ and X², independently of one another, are -COOR or -CN, R representing a C₁ to C₄ -alkyl radical.

3. A process as claimed in either of claims 1 and 2, characterised in that a polar solvent is used concomitantly.

4. A process as claimed in claim 3, characterised in that the polar solvent is a C₁ to C₄ - alcohol.

5. A process as claimed in any one of claims 1 to 4, characterised in that a basic catalyst is used concomitantly.

6. A process as claimed in claim 5, characterised in that the basic catalyst is a metal hydroxide, metal carbonate, metal bicarbonate, a basic ion exchanger or an alcoholate.

7. A process as claimed in claim 5, characterised in that the basic catalyst is an alkali metal hydroxide, alkali metal carbonate, alkali metal bicarbonate or alkali metal alcoholate.

8. A process as claimed in any one of claims 1 to 7, characterised in that the process is carried out batchwise and the reaction temperature is from -30 to 120°C.

9. A process as claimed in any one of claims 1 to 7, characterised in that the process is carried out batchwise and the reaction temperature is from 0 to 60°C.

10. A process as claimed in any one of claims 1 to 7, characterised in that the process is carried out continuously and the reaction temperature is up to 200°C.

11. A process as claimed in any one of claims 1 to 9, characterised in that the process is carried out batchwise by the C-H-acidic compound of the general formula II being added to a suspension of paraformaldehyde in a solvent or solvent mixture.

12. A process as claimed in claim 10, characterised in that, after the reaction is complete, the solid catalyst is separated off or the dissolved catalyst is neutralised and the salt formed is separated off.

13. A process as claimed in any one of claims 1 to 12, characterised in that the bis(hydroxymethyl) compound of the general formula I is further reacted in the reaction mixture in which it is formed, without being isolated.

## Revendications

1. Procédé de préparation de composés bishydroxyméthylés de formule générale : 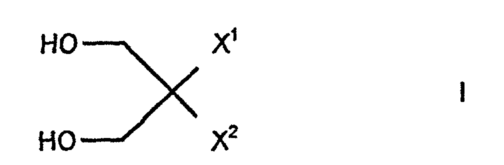
dans laquelle X¹ et X² indépendamment l'un de l'autre, signifient les groupes -COOR, -CONR₂, -CN, -NO₂, -C(OR)=NR ou -COR, dans lesquels R représente de l'hydrogène, un alkyle, un aralkyle, un aryle ou un cycloalkyle ayant respectivement jusqu'à 12 atomes de carbone, avec la restriction que
(i) les deux substituants X¹ et X² ne peuvent pas signifier en même temps -COOH et
(ii) quand les deux substituants X¹ et X² signifient en même temps -CO-R les deux substituants R peuvent signifier ensemble aussi un reste alkylène ayant de 3 à 9 atomes de carbone, par réaction de composés C-H acides de formule générale : 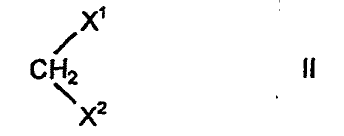
dans laquelle X¹ et X² sont définis comme précédemment, avec du formaldéhyde,
caractérisé en ce qu'
on utilise le formaldéhyde sous forme de p-formaldéhyde et qu'on effectue la réaction en milieu exempt d'eau pour lequel l'acide acétique est exclu.

2. Procédé selon la revendication 1,
caractérisé en ce que
X¹ et X² indépendamment l'un de l'autre représentent -COOR ou -CN dans lequel R signifie un reste alkyle en C₁-C₄.

3. Procédé selon l'une quelconque des revendications 1 à 2,
caractérisé en ce qu'
on utilise conjointement un solvant polaire.

4. Procédé selon la revendication 3,
caractérisé en ce que
le solvant polaire est un alcool en C₁-C₄.

5. Procédé selon l'une quelconque des revendications 1 à 4,
caractérisé en ce qu'
on utilise conjointement un catalyseur basique.

6. Procédé selon la revendication 5,
caractérisé en ce que
le catalyseur basique est un hydroxyde, un carbonate, un hydrogénocarbonate métallique, un échangeur d'ions basique ou un alcoolate.

7. Procédé selon la revendication 5,
caractérisé en ce que
le catalyseur basique est un hydroxyde, un carbonate, un hydrogénocarbonate de métal alcalin ou un alcoolate de métal alcalin.

8. Procédé selon l'une quelconque des revendications 1 à 7,
caractérisé en ce que
le procédé est exécuté graduellement et la température de réaction est de -30 à 120°C.

9. Procédé selon l'une quelconque des revendications 1 à 7,
caractérisé en ce que
le procédé est exécuté graduellement et la température de réaction est de 0 à 60°C.

10. Procédé selon l'une quelconque des revendications 1 à 7,
caractérisé en ce que
le procédé est exécuté graduellement et la température de réaction s'élève jusqu'à 200°C.

11. Procédé selon l'une quelconque des revendications 1 à 9,
caractérisé en ce que
le procédé est exécuté graduellement, dans lequel le composé C-H acide de formule générale 11 est ajouté par doses à une suspension de p-formaldéhyde dans un solvant ou mélange de solvants.

12. Procédé selon la revendication 10,
caractérisé en ce qu'
après achèvement de la réaction, on sépare le catalyseur solide ou on neutralise le catalyseur dissous et on sépare le sel qui s'est formé.

13. Procédé selon l'une quelconque des revendications 1 à 12,
caractérisé en ce que
le composé bishydroxyméthylé de formule générale 1 est mis encore à réagir dans le mélange réactionnel dans lequel il se produit, sans isolement.
